# DEMANDE DE BREVET EUROPEEN

(11) **EP 0 781 562 A2**
(43) Date de publication de la demande: **02.07.1997**
(21) Numéro de dépôt: 96402882.3
(22) Date de dépôt: 24.12.1996
(51) Int. Cl.: A61L 9/01

(54) **Utilisation d'un désodorisant à base d'acide undécylénique ou de dérivés dudit acide pour la désodorisation de papiers, cartons et non-tissés**

(30) Priorité: 26.12.1995 FR 9515477
(71) Demandeur: ELF ATOCHEM S.A., 92800 Puteaux (FR)
(72) Inventeur: Bourson, Lucien, 92270 Bois-Colombes (FR); Caupin, Henri-Jean, 78000 Versailles (FR)

(57) **Abrégé**

L'invention a pour objet l'utilisation d'un désodorisant comprenant l'acide undécylénique, un sel ou un ester alkylique ou polyoxyalkylénique d'acide undécylénique pour la désodorisation des papiers, cartons et non-tissés.

Elle concerne également les papiers, cartons et non-tissés renfermant le désodorisant précité.

## Description

L'invention a pour objet l'utilisation d'un désodorisant comprenant l'acide undécylénique, un sel ou un ester d'acide undécylénique pour désodoriser les papiers, les cartons et les non-tissés.

Dans le domaine de la désodorisation, on connaît déjà des compositions à base d'ester d'acide undécylénique capables d'atténuer certaines odeurs indésirables.

Ainsi, dans DE-A-2 263 509, on propose une composition déodorante complexe comprenant un ester alkylique (C₁-C₄) d'acide undécylénique pour prévenir le développement des micro-organismes responsables des odeurs corporelles chez l'Homme.

Dans DE-A-1 792 467, on décrit une composition destinée à repousser le gibier sauvage qui endommage les cultures à base d'acide undécylénique éventuellement associé entre-autres à des esters d'acide gras insaturé tels que l'undécylénate de méthyle.

Il est également connu d'utiliser des compositions à base d'ester d'acide undécylénique pour désodoriser les boues des stations d'épuration, les lisiers, les aliments pour animaux et les effluents de papeterie (voir FR 2 655 856, FR 2 655 857, FR 2 655 819 et FR 2 694 197).

En matière de supports de nature cellulosique, le besoin est grand de les désodoriser soit parce qu'ils présentent une odeur spécifique marquée, soit parce qu'ils sont destinés à entrer en contact avec des produits malodorants.

La nécessité d'une désodorisation au sens défini ci-avant est manifeste dans le cas des cartons d'emballage et des papiers avant ou après impression dont l'odeur propre est incommodante. Elle apparaît également lorsque l'on souhaite utiliser des papiers ou des non-tissés en tant que support de déodorant, notamment pour filtrer des gaz (climatisation, milieu industriel), des liquides ou des particules (sacs pour aspirateur), recueillir des excréments humains (diapers, couches) ou animaux (litières) ou encore agir en atmosphère confinée (diffuseurs d'ambiance).

Il a maintenant été trouvé que des compositions à base d'acide undécylénique, de sel ou d'ester d'acide undécylénique s'avèrent particulièrement efficaces pour désodoriser les papiers, les cartons et les non-tissés.

Plus précisément, l'invention concerne l'utilisation d'une substance absorbante d'odeurs caractérisée en ce que ladite substance comprend l'acide undécylénique, un sel ou un ester alkylique ou polyoxyalkylénique d'acide undécylénique.

Parmi ces produits, l'invention concerne tout particulièrement les sels métalliques, les esters alkyliques renfermant de 1 à 12 atomes de carbone et les esters de polyoxyéthylène, polyoxypropylène et poly(oxyéthylène)(oxypropylène) dudit acide undécylénique. Elle concerne plus précisément les undécylénates de Na, K, Zn, Ca et Cu, de méthyle, d'éthyle, de propyle, d'hexyle, de décyle ainsi que les esters polyoxyalkyléniques renfermant de 2 à 20 motifs oxyalkylène.

La substance absorbante utilisée conformément à l'invention peut être constituée d'un seul des produits précités ou d'un mélange desdits produits, ce ou ces produits pouvant eux-mêmes être mis en oeuvre tels quels ou sous forme de solution, suspension ou émulsion, ou encore sous forme adsorbée sur un support notamment solide tel que l'argile, les zéolithes ou les diatomées.

D'une manière générale, l'acide undécylénique, les sels ou esters undécyléniques sont efficaces à faible dose, par exemple de l'ordre de 0,05 à 10 % en poids, et de préférence 0,05 à 3, par rapport au support à traiter.

On peut naturellement former des compositions comprenant l'acide undécylénique, le ou les sels ou esters undécyléniques précités et des additifs destinés notamment à améliorer les propriétés mécaniques, d'aspect, de résistance aux graisses, à la pénétration de l'eau, l'état de surface....

L'utilisation de compositions à base des composés précités est généralement réalisée sur tout type de support comprenant des fibres cellulosiques tel que les papiers, les cartons et les non-tissés. Elle est tout particulièrement recommandée pour désodoriser les papiers spéciaux. A titre d'illustration de ces papiers, on peut citer les papiers à cigarette, pour filtre (café, aspirateur ou à usage industriel), pour condensateur, pour joint, sulfurisé ou simili sulfurisé, à dessin, mural, pour enduction de silicone ou d'auto-adhésif, fiduciaires, pour géographie, buvard, pour abrasifs, autocopiant, pour essuyage, à usage médical et pour emballage de pièces métalliques ou d'aliments pour animaux.

Les exemples qui suivent permettent d'illustrer l'invention.

### EXEMPLE 1

On prépare de manière conventionnelle un papier non couché pour impression offset de grammage 70 m²/g à partir de la composition suivante (en % en poids) :

| | |
|---|---|
| Pâte kraft blanchie de résineux (KBR) | 30 |
| Pâte kraft blanchie de feuillus (KBF) | 70 |
| CaCO₃ | 15 (par rapport aux fibres) |
| AKD | 1 |
| Amidon cationique | 0,4 (par rapport à la composition) |
| P.A.A. | 200 g/t de papier |
| Undécylénate de méthyle | 0,01 à 2 |

Sur les deux faces du papier ainsi obtenu, on dépose à la size press de l'amidon oxydé (3 g/m²/face).

Le papier est imprimé à l'aide d'une encre offset (tirant gradué n°5; LORIEUX).

Le degré de perception olfactive du papier imprimé est évalué par un groupe de six personnes qui attribuent une note variant de 0 à 6, les valeurs extrêmes 0 et 6 signifiant respectivement : absence d'odeur d'encre et odeur originelle de l'encre.

Les résultats sont présentés ci-après par comparaison avec un papier témoin ne contenant pas d'undécylénate de méthyle.

| | | | | | |
|---|---|---|---|---|---|
| Undécylénate de méthyle (% en poids) | 0,01 | 0,05 | 0,5 | 2 | Témoin |
| Note | 6 | 4 | 2 | 1 | 6 |

### EXEMPLE 2

On dépose une émulsion aqueuse contenant 10 % en poids d'undécylénate de méthyle (MASKOD MNS 01-10; EC11) à la size press sur un papier non couché (70 m²/g).

Le papier est imprimé à l'aide d'une encre offset (tirant gradué n°5; LORIEUX). Le degré de perception olfactive du papier imprimé est mesuré dans les conditions de l'exemple 1 par comparaison avec un papier témoin sans undécylénate de méthyle.

| | | | | | |
|---|---|---|---|---|---|
| Undécylénate de méthyle (% en poids) | 0,01 | 0,05 | 0,5 | 2 | Témoin |
| Note | 5 | 3 | 1 | 0 | 6 |

### EXEMPLE 3

On prépare un papier destiné à l'emballage d'aliments secs (granulés) pour animaux à partir d'une pâte chimique blanchie (grammage 80 m²/g) et collage en milieu neutre à l'aide d'alkyl-cétène dimères (Cobb 60 = 18 g/m²). Le papier obtenu est rendu imperméable aux graisses par un traitement avec du FORAPERLE 321 (ELF ATOCHEM S.A.) et imprégné en size press avec une émulsion aqueuse contenant 10 % en poids d'undécylénate de méthyle (MASKOD MNS 01-10; EC11) et 0,2 % en poids de carboxyméthylcellulose.

Le papier ainsi obtenu contient 3 g/m² d'undécylénate de méthyle.

A l'aide de ce papier, on emballe 100 g de granulés et on dispose l'ensemble dans un bocal, lequel bocal est placé dans une étuve à 50°C pendant 24 heures.

A l'ouverture du bocal, on ne constate aucune odeur alors qu'un bocal témoin contenant des granulés emballés dans le même papier sans undécylénate de méthyle présente une très forte odeur d'aliment.

Les granulés emballés dans le papier traité sont parfaitement acceptés par les animaux.

### EXEMPLE 4

On prépare un voile de non-tissé (60 m²/g) à partir d'une composition fibreuse contenant 50 % en poids de pâte chimique et 50 % en poids de fibres de polypropylène.

Le voile obtenu est imprégné en size press avec une émulsion aqueuse contenant 10 % en poids d'undécylénate de méthyle (MASKOD MNS 01-10; EC11) et 0,1 % en poids de carboxyméthylcellulose. Ce voile imprégné contient 1g/m² d'undécylénate de méthyle.

Le voile est placé dans les chaussures de marche de 6 utilisateurs. Après 10 jours d'utilisation, on ne perçoit aucune odeur.

## Revendications

1. Utilisation d'un désodorisant comprenant l'acide undécylénique, un sel ou un ester alkylique ou polyoxyalkylénique d'acide undécylénique pour la désodorisation des papiers, cartons et non tissés.

2. Utilisation selon la revendication 1 caractérisée en ce que le sel est choisi parmi les sels métalliques.

3. Utilisation selon la revendication 2 caractérisée en ce que le sel est l'undécylénate de Na, K, Zn, Ca ou Cu.

4. Utilisation selon la revendication 1 caractérisée en ce que l'ester est choisi parmi les esters alkyliques renfermant de 1 à 12 atomes de carbone et les esters de polyoxyéthylène, polyoxypropylène et poly(oxyéthylène)(oxypropylène) de l'acide undécylénique.

5. Papier contenant un désodorisant selon l'une des revendications 1 à 4.

6. Carton contenant un désodorisant selon l'une des revendications 1 à 4.

7. Non-tissé contenant un désodorisant selon l'une des revendications 1 à 4.
